# EUROPEAN PATENT APPLICATION

(11) **EP 1 029 913 A1**
(43) Date of publication of application: **23.08.2000**
(21) Application number: 98928612.5
(22) Date of filing: 22.06.1998
(51) Int. Cl.: C12M 1/00

(54) **AGITATING INCUBATOR, DISPENSER INCLUDING THE SAME, AND DISPENSING METHOD**

(30) Priority: 16.10.1997 JP 28388397; 19.03.1998 JP 7047598; 25.03.1998 JP 7746398
(71) Applicant: NITTETSU MINING CO., LTD., Tokyo 100 (JP); SANKYO COMPANY LIMITED, Tokyo 140-0005 (JP)
(72) Inventor: INABA, Kazuhiro, Nittetsu Mining Co., Ltd., Nishitama-gun (JP); WAKASA, Toshiyuki, Nittetsu Mining Co., Ltd., Nishitama-gun, Tokyo (JP); SHIRATORI, Mamoru, Sankyo Company, Limited, Tokyo 140-0005 (JP); ICHIKAWA, Masato, Sankyo Company, Limited, Tokyo 140-0005 (JP)
(74) Representative: Grünecker, Kinkeldey, Stockmair & Schwanhäusser Anwaltssozietät
(86) International application number: JP9802771
(87) International publication number: WO9920737

(57) **Abstract**

A medium dispenser capable of preventing dripping of a solution and pulsating discharge causing variation of the quantity of dispensed medium and foams, and making it possible to reliably supply the medium at a suitable temperature while preventing local gelling of the medium and contamination with miscellaneous bacteria, wherein: a medium return tube (14) for returning the medium to a medium container (3) is connected to and branched from an intermediate part of a medium feed tube (11) acting as a medium flow passage for guiding the medium (2) from the medium container (3) to a nozzle (7) to allow the medium to circulate while it is kept warm at the time of non-dispensing, while the portion of the medium flow passage extending from the branching point of the medium return tube (14) at the medium feed tube (11) to the nozzle (7) is heated as a whole with auxiliary heating means (16); at the time of dispensing a suitable quantity of the medium is supplied through throttle portions (50, 51) disposed at least two positions of the tube (11) and having a diameter smaller than the diameter of the other portions of the passage; and a first stirrer (5a) inside the medium container (3) and a second stirrer (5b) inside a heating tank (6) are used to provide a uniform stirring and heat insulation inside the medium container.

## Description

### Technical Field

The present invention relates to a stirring and heat-retaining apparatus for retaining the temperature of a medium solution such as an agar medium which may be used in various kinds of microbial tests, while stirring the medium solution, and also to a dispensing apparatus and a dispensing method which use such a stirring and heat-retaining apparatus and supply a predetermined medium solution to a large number of containers. Particularly, the invention relates to a dispensing apparatus and a dispensing method which dispense a predetermined amount of a medium solution (medium) for an agar medium.

### Background Art

Generally, a medium such as an agar medium is widely used in: microbial tests in, for example, the medicinal industry and relating to GMP validation (Good Manufacturing Practice Validation), such as a axenic test, a bacteria limiting test, an environmental falling bacteria test, a measurement of the titer (efficacy) of an antibiotic, and a body fluid concentration measurement; microbial tests in, for example, the food industry, and relating to a countermeasure of preventing contamination due to destructive fungus based on Haccp (Hazard Analysis Critical Control Point), such as a viable cell count test, and a fungus count test.

Such various kinds of microbial tests are roughly classified into a qualitative test and a quantitative test. In a quantitative test, an agar medium is almost always used.

Usually, after a medium is poured into a laboratory dish and then spread into a flat shape of a uniform thickness, the medium is used in a test. Depending on the kind of a test, a medium which has been poured into a laboratory dish may be spread into a flat shape to form a base medium, and a medium may be further dispensed onto the solidified base medium to form a multilayer medium.

When a medium is to be poured into a laboratory dish, it is a matter of course that a correct amount of the medium must be poured, and other many severe conditions are imposed. Therefore, skills such as those in a manual operation are required. In such various kinds of tests, when the sampling number of a specimen is large and plural types of media are used, a very long time period is required for sampling the specimen and dispensing the media. Furthermore, the service conditions of a medium are varied depending on the kind of a test. For example, a medium is supplied in an axenic condition to a specimen into which bacteria are added, or bacteria are added to a medium itself. In order to enhance the test accuracy, the work of dispensing a medium must be performed in a sterilized room or the like.

Moreover, it is very important to adequately manage the temperature of a medium during pouring so as to prevent local gelation from occurring, and to prevent air bubbles or the like from entering the medium.

In a microbial test, for example, it is indispensable to adequately manage the temperature of a medium to prevent various kinds of bacteria which are test objects, from dying. In dispensation to a laboratory dish or the like, when the temperature of a medium is not kept to be 50°C or lower, bacteria may die. Generally, the solidification temperature of a medium is higher than room temperature (for example, 24°C). In the case of a solid medium, the medium starts to solidify at 38°C, and is completely solidified at 36°C.

Before dispensation to a laboratory dish, therefore, a medium must be heated to the solidification temperature or higher, to be kept in a liquid state. Otherwise, the medium cannot be uniformly spread in a laboratory dish into which the medium is poured. When the temperature is excessively higher, however, there arises a trouble in that a very long time period is required for a medium to solidify after dispensation, or that bacteria or microorganisms which are to be used in a test die.

That is, it is important that a medium is adjusted and managed to an adequate temperature which is higher than the solidification temperature and not higher than required, and the operation of pouring the medium into a laboratory dish is then rapidly completed.

When air bubbles enter a medium, moreover, there arises a fear that, in the case where the air bubbles are minute, the air bubbles may be erroneously recognized as bacteria during measurement. In order to prevent the measurement accuracy from being reduced by erroneous recognition, it is important to prevent air bubbles from entering a medium.

Such a temperature management of a medium is very important in works of mixing and diluting a specimen with the medium, and uniformly spreading the medium.

Under such circumstances, an apparatus for automatically performing sampling of a specimen and the work of dispensing a medium is disclosed in, for example, Japanese Unexamined Patent Publications (Kokai) Nos. Hei. 5-153961, Hei. 4-248980, and Hei. 3-49676.

As a configuration of such an apparatus, for example, various medium dispensing apparatuses comprising: a medium container which stores a medium; stirring means for stirring the medium in the medium container; heating means for adjustingly heating the medium in the medium container to a desired temperature; a nozzle for pouring the medium into a laboratory dish serving as the dispensing container; a medium passage which guides the medium in the medium container to the nozzle; a dispensing pump which sequentially sends out the medium in the medium container to the medium passage; and a controller which controls operations of the stirring means, the heating adjusting means, and the dispensing pump have been proposed.

On the other hand, a temperature management is essential also in another analysis inspection. In an analysis inspection which is employed in odor measurement for industrial effluent, for example, retention at a fixed temperature (for example, 25°C) or lower is required. When the industrial effluent contains a large amount of suspending materials, a stirring work for about one minute is further required. The odor measurement is conducted after such procedures.

From the above, in storage of a liquid which is to be analyzed and which has not yet been inspected, such as a medium, stirring and heat retention must be conducted at the same time so that uniformalization and temperature management of the medium are simultaneously performed.

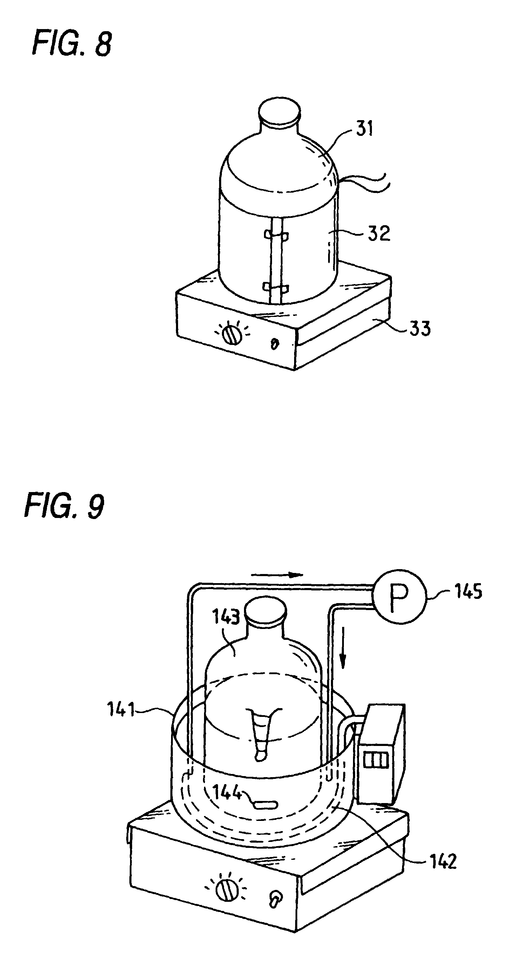

Medium temperature management for a microbial inspection will be exemplarily described. As a method of stirring and retaining the temperature of a medium in a medium container for storing a medium, a method is known in which a band heater 32 that is closely contacted with the outer peripheral face of a medium container 31 as shown in Fig 8 is connected to an external power source to be heated, thereby raising the temperature of the medium container 31 which is contacted with the band heater 32, to heat a medium in the medium container, and, on the other hand, a magnetic stirrer 33 that is disposed below the medium container 31 rotates a stirring rotor configured by permanent magnets (not shown) and disposed in the medium container, whereby the medium is stirred.

In the magnetic stirrer 33, the magnets are placed on the ends of a rod member, respectively, and the rod member is rotated with being pivotally supported at the center so that the magnets at the ends move along a substantially same circular orbit. In accordance with the change in magnetic field (polarity) of the surroundings due to the rotation of the rod member, magnetic attractive and repulsive forces are produced in the stirring rotor so as to rotate the stirring rotor.

In a configuration wherein, as shown in Fig. 9, a heater 142 is submerged in a heating bath 141 filled with water and a medium container 143 is placed in the heating bath 141, another method is performed in the following manner. The heater 142 is heated to raise the temperature of the water in the heating bath 141, whereby the temperature of a medium in the medium container 143 is managed. On the other hand, in the same manner as the configuration shown in Fig. 8, the medium is stirred by a stirring rotor 144 in the medium container 143.

In such a conventional dispensing apparatus, when a medium is to be supplied from a medium container to a laboratory dish by liquid supply driving means such as a pump, the supply amount and the supply timing are set by opening and closing of valve means (for example, a control valve) disposed in the liquid supply driving means. During a stoppage of the supply or at the moment when the supply is stopped, the medium may drip from the dispensing nozzle, with the result that the supply amount is varied or, in the next dispersion, air bubbles are formed in the medium by liquid dripping. When the medium is ejected from the dispensing nozzle, moreover, the medium ejection is pulsated by the affection of pulsation of the liquid supply driving means and the medium at the nozzle end has a recessed shape, thereby causing other problems in that the dispensation becomes unstable, and that bubbles are easily formed in the medium. In the case where the air bubbles are minute, there is a further problem in that the air bubbles may be erroneously recognized as bacteria during measurement.

At the start of the operation, or when the dispensation is conducted at a long interval, the temperature of the medium passage from the medium container to the nozzle is considerably lower than that of the medium the temperature of which is retained. Therefore, the temperature of the medium which is supplied through the medium passage is lowered by heat absorption due to the medium passage, with the result that the dispensation is hardly conducted at an adequate temperature.

In order to cope with the problems, a method may be employed in which, at the start of the operation, a heated medium is kept to flow until the medium passage from the medium container to the nozzle is heated to an adequate temperature. In such a countermeasure method, however, a large amount of the medium is wasted.

In order to prevent air bubbles and various bacteria from entering, a process of disposing of a medium which remains in the nozzle or the like to be exposed to the outside air is conducted immediately before the start of the next dispensing operation. Also in this case, it is difficult to control the discharge amount of the medium to a minimum level, only by the operation control based on the dispensing pump. Therefore, there arises a further problem in that the medium is wastefully consumed.

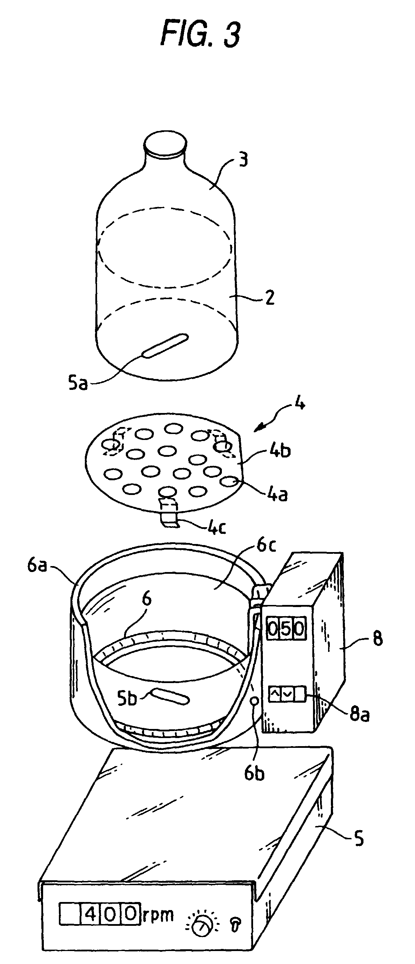

Problems of a stirring and heat-retaining apparatus will be discussed. Fig. 3 shows a conventional medium stirring and heat-retaining apparatus. In the apparatus, when the liquid level of the medium in the medium container 31 is lower than the height the heating region of the band heater 32, a local phenomenon of no-water heating by the band heater 32 may occur in the heating zone of the wall face of the medium container 31 and above the medium level, and the medium in the no-water burned portion may be locally gelled. By this degradation, moreover, the temperature of the medium is made uneven, and hence it is difficult to finely set the medium temperature. Because the band heater 32 is wound around the barrel portion (peripheral face) of the medium container 31, the medium container must be formed into a straight pipe-like shape, thereby causing a further problem in that the shape of the medium container is restricted.

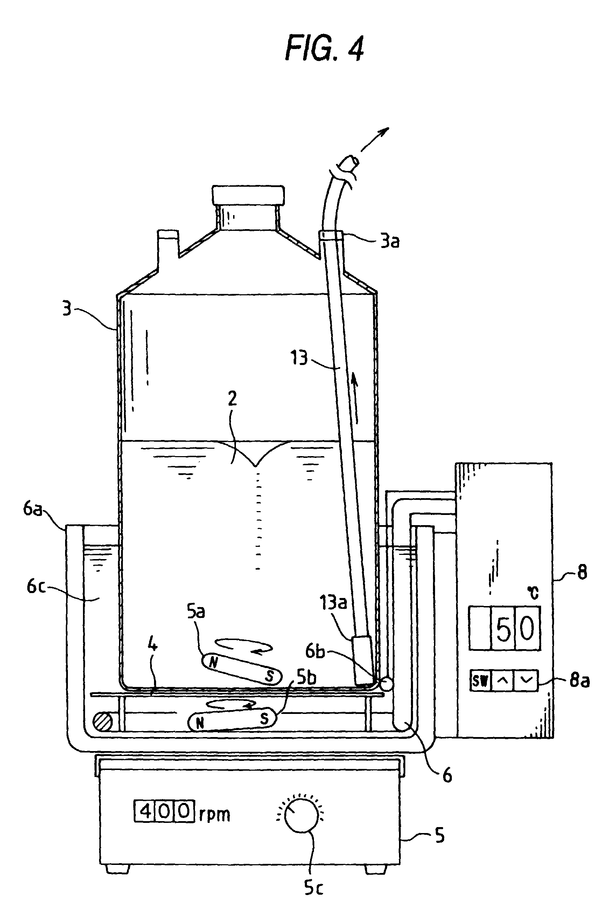

In another prior art medium stirring and heat-retaining apparatus shown in Fig. 4, circulation of water in a heating bath 41 is conducted only in the form of natural circulation, and hence temperature unevenness occurs in the upper and lower portions of the heating bath 41, so that the bath temperature cannot be finely set. As a result, it is difficult to finely manage the medium temperature. To comply with this, for example, it may be contemplated that a pump 45 for circulating the bath water is disposed to forcedly circulate the water in the heating bath. However, this produces new problems such as that the apparatus itself becomes expensive, and that maintenance such as cleaning of the pump, and management for sanitation such as sterility of the bath water are hardly conducted.

As described above, any stirring and heat-retaining apparatus has a problem in that the viscosity, the temperature, and the like of a medium cannot be uniformalized unless the medium is sufficiently stirred by using a stirring apparatus consisting of a magnetic stirrer, a rotor, etc.

In the prior art, namely, there is no stirring and heat-retaining apparatus which satisfies simultaneously all of the conditions of a medium including the heat-retaining (heating) property and the stirring property and indicated in following (1) to (5) :
(1) The temperature of a medium is enabled to be retained (heated) irrespective of the shape of a medium container.
(2) Even when the internal capacity of a medium container is changed, the temperature of a medium is enabled to be constantly retained (heated).
(3) A structure of a high heat retaining property in which the loss of thermal energy to the outside is reduced as much as possible is attained (from the viewpoint of energy saving).
(4) A medium in a medium container is prevented from being locally gelled.
(5) The viscosity of a medium in a medium container is kept constant.

Therefore, it is an object of the invention to solve the above-discussed problems, and provide a medium dispensing apparatus and a dispensing method in which, even when dispensation amount of a medium is small, variation of the dispensation amount of the medium, liquid dripping which produces air bubbles, and pulsated ejection are prevented from occurring, air bubbles and various bacteria are surely prevented from entering by a minimum disposal amount of the medium, and the whole region of a dispensation passage is stably maintained to an adequate temperature to surely realize dispensation of the medium at the adequate temperature.

It is another object of the invention to provide a medium stirring and heat-retaining apparatus which can satisfy simultaneously the conditions (1) to (5).

### Disclosure of Invention

The medium dispensing apparatus and the dispensing method of the invention are configured in the following manner.
1. A medium dispensing apparatus which pours a medium 2 stored in a medium container 3 having a stirring and heat-retaining apparatus, into a dispensing container 21, wherein the medium dispensing apparatus comprises:
   a medium sending passage 11 serving as a medium passage which guides the medium 2 in the medium container 3 to a nozzle 7 for pouring the medium into the dispensing container 21;
   a medium returning passage 14 serving as a medium passage one end of which is branched from a midway of the medium sending passage 11, and another end of which is connected to the medium container 3 to return the medium 2 to the medium container 3;
   a dispensing pump 15 which is disposed upstream from a branching position C between the medium sending passage 11 and the medium returning passage 14, and which sends out the medium 2 in the medium container 3 to the medium sending passage 11;
   a first control valve 18 which is disposed in a midway of the medium sending passage 11 and downstream from the branching position C, and which opens and closes the passage of the medium sending passage 11;
   a second control valve 19 which is disposed in a midway of the medium returning passage 14 and downstream from the branching position C, and which opens and closes the medium returning passage 14;
   auxiliary heating means 16 for, in a zone which is downstream from the branching position C of the medium sending passage 11, adjustingly heating the medium in the medium sending passage 11 to a desired temperature; and
   a controller which controls operations of the dispensing pump 15, the auxiliary heating means 16, and the first and second control valves 18 and 19.
2. A medium dispensing apparatus according to paragraph 1, wherein the auxiliary heating means 16 is an infrared lamp.
3. A medium dispensing apparatus according to paragraph 1 or 2, wherein a filter 27 which makes air to be supplemented into the medium container 3 into an axenic condition is disposed at an upper position of the medium container 3.
4. A medium dispensing apparatus according to paragraph 1, wherein, in the medium sending passage 11 which is downstream from the first control valve 18, throttle portions 50 and 51 of a diameter which is smaller than a diameter of another portion of the medium sending passage 11 are disposed in two or more positions.
5. A medium dispensing apparatus according to paragraph 4, wherein at least a portion of the medium sending passage 11 is made of an elastic material, and the portion made of the elastic material is sandwiched by pressing means 40, whereby the throttle portions 50 and 51 are formed.
6. A medium dispensing apparatus according to paragraph 5, wherein the pressing means 40 is configured so that throttle diameters of the throttle portions 50 and 51 are adjustable.
7. A medium dispensing apparatus according to paragraph 1 or 4, wherein the medium stirring and heat-retaining apparatus comprises: a heating bath 6a which stores a liquid heat transfer medium in which the medium container 3 is to be immersed, the bath having heating means S for heating the heat transfer medium to retain the temperature of the medium in the medium container 3; a pedestal 4 on which the medium container 3 is placed in the heating bath 6a; a first stirrer 5a which is immersibly disposed for stirring the medium in the medium container 3; a second stirrer 5b which is submergedly disposed on a bottom face of the heating bath 6a; and magnetic stirring means for rotating simultaneously the first stirrer 5a and the second stirrer 5b, the pedestal 4 being disposed at a height which is above the second stirrer 5b and at which rotation of at least the second stirrer 5b is not obstructed.
8. A dispensing method which uses the medium dispensing apparatus according to paragraph 1 or 4 to pour a predetermined amount of the medium into the dispensing container, wherein,
   during an operation of waiting for dispensation, the first control valve 18 is closed, and the second control valve 19 is opened; in the medium sending passage 11 from the medium container 3 to the branching position C, and the medium returning passage 14, the medium 2 is circulated by the dispensing pump 15 to hold the medium to an adequate temperature; and, in the medium sending passage 11 from the branching position C to the nozzle 7, the medium 2 is held to an adequate temperature by heating conducted by the auxiliary heating means 16.
9. A dispensing method which uses the medium dispensing apparatus according to paragraph 1 or 4 to pour a predetermined amount of the medium into the dispensing container, wherein,
   during an operation of waiting for dispensation, a state where the nozzle 7 is retracted to a waiting position B separated from a dispensing position A is held; and, at a start of a dispensing operation, the dispensing pump 15 is operated for a predetermined time period immediately before the start under a state where the second control valve 19 is closed and the first control valve 18 is opened, to discharge a medium which is at a tip end of the nozzle 7 and inadequate in quality, at the waiting position B, and the nozzle 7 is thereafter advanced to the predetermined dispensing position A to conduct dispensation.
10. A dispensing method which uses the medium dispensing apparatus according to paragraph 3 to pour a predetermined amount of the medium into the dispensing container, wherein,
   in the medium sending passage 11 from the medium container 3 to the branching position C, and the medium returning passage 14, during an operation of waiting for dispensation, the medium 2 in the medium container 3 is circulated by the dispensing pump 15 while the first control valve 18 is closed and the second control valve 19 is opened, to hold the medium 2 in an axenic condition; and, during dispensation of the medium 2, air in an axenic condition is supplemented into the medium container 3 via the filter 27.
11. A medium dispensing apparatus comprising:
   a dispensing nozzle 7 which supplies a specific amount of a medium 2 to a predetermined container;
   liquid supply driving means for sending the medium 2 to the dispensing nozzle 7;
   a medium sending passage 11 which forms a hermetically sealed liquid passage between the dispensing nozzle 7 and the liquid supply driving means; and
   valve means for opening and closing the medium sending passage 11 in order to control an amount of the medium 2 supplied to the container,
      wherein throttle portions 50 and 51 of a diameter which is smaller than a diameter of another portion of the medium sending passage 11 being disposed in two or more positions of the medium sending passage 11.
12. A medium dispensing apparatus according to paragraph 11, wherein at least a portion of the medium sending passage 11 is made of an elastic material, and the portion made of the elastic material is sandwiched by pressing means 40, whereby the throttle portions 50 and 51 are formed.
13. A medium dispensing apparatus according to paragraph 12, wherein the pressing means 40 is configured so that throttle diameters of the throttle portions 50 and 51 are adjustable.
14. A medium stirring and heat-retaining apparatus comprising:
   a heating bath 6a which stores a liquid heat transfer medium in which a medium container 3 containing a medium is to be immersed;
   heating means 6, disposed in the heating bath 6a, for heating the heat transfer medium to retain the temperature of the medium in the medium container 3;
   a pedestal 4 on which the medium container 3 is placed in the heating bath 6a;
   a first stirrer 5a which is immersibly disposed for stirring the medium in the medium container 3;
   a second stirrer 5b which is submergedly disposed on a bottom face of the heating bath 6a; and
   magnetic stirring means for rotating simultaneously the first stirrer 5a and the second stirrer 5b,
      wherein the pedestal 4 being disposed at a height which is above the second stirrer 5b and at which rotation of at least the second stirrer is not obstructed.

### Brief Description of Drawings

Fig. 1 is a schematic view of an embodiment of the medium dispensing apparatus of the invention;
Fig. 2 is a flowchart showing procedures of a medium dispensing method which is implemented by the embodiment of Fig. 1;
Fig. 3 is an assembly view of a medium stirring and heat-retaining apparatus of an embodiment of the invention;
Fig. 4 is a view illustrating the operation of the medium stirring and heat-retaining apparatus of the embodiment of the invention;
Fig. 5 is a schematic view of a second embodiment of the medium dispensing apparatus of the invention;
Fig. 6 is a longitudinal section view taken along a liquid passage in a portion where pressing means shown in Fig. 5 is disposed;
Fig. 7 is a section view taken along the line X-X in Fig. 6;
Fig. 8 is a view showing the configuration of a conventional stirring and heat-retaining apparatus; and
Fig. 9 is a view showing the configuration of another conventional stirring and heat-retaining apparatus.

### Best Mode for Carrying Out the Invention

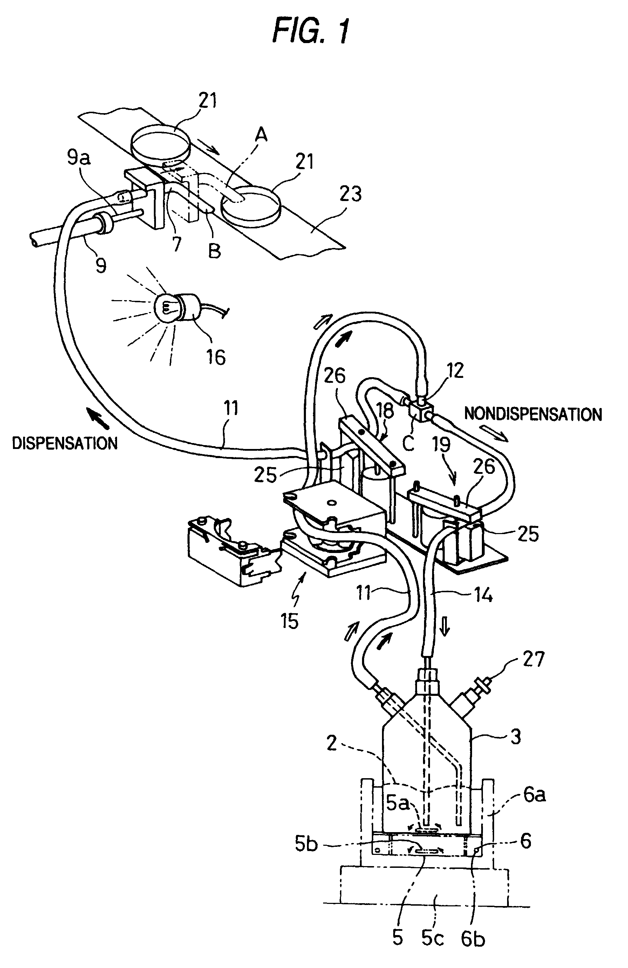

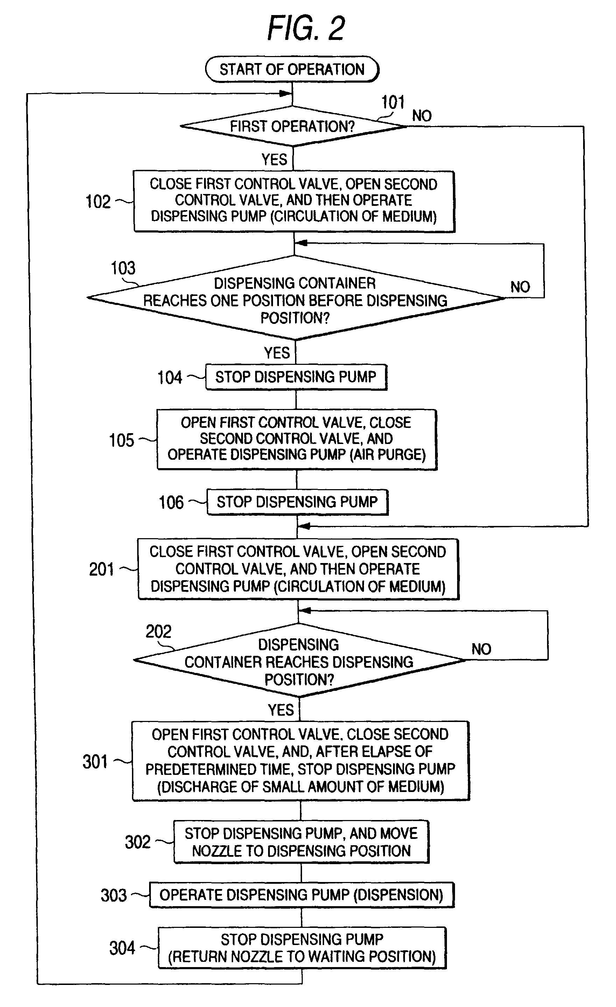

Hereinafter, an embodiment of the medium dispensing apparatus and the dispensing method of the invention will be described in detail with reference to Figs. 1 and 2.

Fig. 1 is a schematic view of an embodiment of the medium dispensing apparatus of the invention, and Fig. 2 is a flowchart showing procedures of a medium dispensing method which is implemented by the embodiment having this configuration.

As shown in Fig. 1, the medium dispensing apparatus 1 of the embodiment is configured by:
a medium container 3 which stores a medium 2 such as an agar medium;
stirring means 5 for stirring the medium 2 in the medium container 3;
heating means 6 for adjustingly heating the medium 2 in the medium container 3 to a desired temperature;
a nozzle 7 for pouring the medium 2 into the dispensing container;
nozzle moving means 9 for moving the nozzle 7 between a predetermined dispensing position A and a waiting position B separated from the dispensing position A;
a medium sending tube 11 serving as a medium sending passage that is a medium passage which guides the medium 2 in the medium container 3 to the nozzle 7;
a trifurcated pipe joint 12 for branching connection which is placed in a midway of the medium sending tube 11;
a medium returning tube 14 serving as a medium returning passage that is a medium passage one end of which is branched from a midway of the medium sending tube 11 via the trifurcated pipe joint 12, and another end of which is connected to the medium container 3 to return the medium 2 to the medium container 3;
a dispensing pump 15 which is disposed on the medium sending tube 11 and upstream from a branching position C (i.e., the position of the trifurcated pipe joint 12) of the medium sending tube 14, and which sequentially sends out the medium 2 in the medium container 3 to the medium sending tube 11;
auxiliary heating means 16 for, in a zone which is downstream from the branching position C of the medium sending tube 11, adjustingly heating the medium 2 in the medium sending tube 11 to a desired temperature;
a first control valve 18 which is disposed in the medium sending tube 11 and downstream from the branching position C, and which opens and closes the passage of the medium sending tube 11;
a second control valve 19 which is disposed in the medium returning tube 14 and downstream from the branching position C, and which opens and closes the medium returning tube 14;
container transporting means 23 for sending in and out a laboratory dish 21 that is a dispensing container to which the medium 2 is dispensed from the nozzle 7, to the dispensing position A along a predetermined route; and a controller (not shown) which controls operations of these components.

In the heating means 6, heating is conducted by a ring-like heater 6b disposed on an inner bottom portion of a heating bath 6a in which the medium container 3 is to be bathed. The quantity of heat generated by the ring-like heater 6b is set with monitoring the temperature of the medium container 3 or the like so that the medium 2 in the medium container 3 has an adequate temperature, and controlled by a temperature adjusting device.

The stirring means 5 is configured by: a medium stirring rotor 5a which is submerged in the medium container 3; a medium stirring rotor 5b which is submergedly disposed on the bottom portion of the heating bath 6a; and a magnetic controller 5c which controls simultaneously rotations of both the rotors 5a and 5b by non-contact magnetic means. The stirring strength is controlled by controlling the rotational amounts of the rotors 5a and 5b.

The heating means 6 and the stirring means 5 which act on the medium container 3 will be described in detail later.

In the embodiment, the nozzle moving means 9 is a linear fluid-pressure cylinder in which the tip end of an extendable and retractable rod 9a is coupled to the nozzle

As the tubes 11 and 14, a silicone tube which is excellent in elasticity and durability is used.

The dispensing pump 15 has a known structure which pushes out the medium in the tube 11 by means of a group of rollers that are rotated so as to collapse the tube. The ejection amount of the medium is adjusted by controlling the rotational amount of a table supporting the roller group. In a passage system in which axenicity is important, particularly, a dispensing pump having such a configuration is effective in preventing various bacteria from entering.

In the embodiment, an infrared lamp is used as the auxiliary heating means 16. With respect to the infrared lamp, the quantity of generated heat, the separation distance, and the like are previously set so that the medium sending tube 11 has an adequate temperature. As compared with other heating means, an infrared lamp can conduct heating which is substantially uniform in the whole, without heating a local portion to a high temperature. Therefore, an infrared lamp is effective particularly in the case such as that of the embodiment where microorganisms contained in a medium die at a predetermined temperature and hence a severe temperature management is required.

In each of the control valves 18 and 19, a pair of pinching members 25 and 26 are disposed so as to extendable and retractable in a radial direction of the tube, and the passage is closed by pressing the tube by the pair of pinching members 25 and 26. In the figure, the first control valve 18 is opened, and the second control valve 19 is closed. The pinching members 25 and 26 are driven by actuators such as solenoids.

The container transporting means 23 uses travelling means or a moving mechanism which is known, to transport laboratory dishes 21 at predetermined intervals and speed. Usually, the means is configured so that, when a laboratory dish 21 reaches the dispensing position A, the transportation of the laboratory dish 21 is temporarily stopped until the dispensing work is ended, and the medium is uniformly spread by rotating the laboratory dish 21 after dispensation while inclining the laboratory dish. In the case where the transportation speed is low and the dispensing work can be conducted while transportation is performed, alternatively, the means may be configured so that dispensation is conducted without stopping the laboratory dish 21 at the dispensing position A.

On the other hand, air in an axenic condition is always supplemented into the container 3 via a disposal filter 27 in which a commercially available membrane filter and a holder are integrated together. By the disposal filter 27, the outside air which is supplemented into the medium container 3 during the medium dispensation is sterilized, so that the medium 2 can be prevented from being directly contacted with the outside air. As a result, the medium 2 can be always held in an axenic condition.

In the medium dispensing apparatus 1, specifically, the controller which is not shown controls operations of the stirring means 5, the heating means 6, the nozzle moving means 9, the dispensing pump 15, the auxiliary heating means 16, the first and second control valves 19, the container transporting means 23, and the like, and controls the operations of the components in the procedure shown in Fig. 2, thereby implementing the medium dispensing method of the invention. Namely, the medium dispensing method of the invention is implemented by the controller which performs the operation control of the medium dispensing apparatus 1.

Hereinafter, the procedure of the dispensing process which is realized by the controller will, be described with reference to Fig. 2.

At the start of the operation of the medium dispensing apparatus 1, it is judged in step 101 whether the next dispensing operation is a first operation after activation of the apparatus or not. If it is judged that the operation is a second or later operation, the control advances to step 201.

When a power switch of the medium dispensing apparatus 1 is pressed, the stirring means 5, the heating means 6, and the auxiliary heating means 16 are automatically started to operate. After elapse of a predetermined time period, transportation by the container transporting means 23 is started. Alternatively, the start of transportation by the container transporting means 23 may be instructed through a dedicated switch.

If it is judged in step 101 that the operation is first operation after activation of the apparatus, a preparation operation consisting of following steps 102 to 106 is conducted, and the control then advances to step 201.

In step 102, the first control valve 18 is closed and the second control valve 19 is opened, and the dispensing pump 15 is then operated to circulate the medium 2 in the medium container 3 through a circulation passage which is formed by a zone from the medium container 3 to the branching position C of the medium sending tube 11, and the medium returning passage 14.

In step 103, the transportation state of the laboratory dishes 21 by the container transporting means 23 is monitored, and, based on detection by a position sensor (not shown) or the like, it is judged whether the leading laboratory dish 21 reaches one position before the dispensing position A or not. If the laboratory dish reaches the position, the operation of the dispensing pump 15 is stopped in next step 104, and, in further next step 105, the first control valve 18 is opened and the second control valve 19 is switched to a closed state, and the dispensing pump 15 is again operated. The operation of step 105 is conducted in order to discharge a old medium remaining in the zone from the branching position C to the nozzle 7, and discharge air which has entered the passage in the zone from the branching position C to the nozzle 7 during the stop of the operation of the apparatus.

After the discharge of the remaining medium of an inadequate quality and the air discharge are completed in step 105, the control advances to next step 106 to stop the dispensing pump 15, and then transfers to next step 201.

In step 201, an operation is conducted in which, in preparation for an actual dispensing operation, the medium 2 in the medium container 3 and the circulation passage is held to an adequate temperature and the control waits. Specifically, in a state where the first control valve 18 is closed and the second control valve 19 is opened, the dispensing pump 15 is operated to circulate the medium 2 in the medium container 3. This circulation of the medium causes the medium 2 which is heated in the medium container 3, to circulate while transferring heat to the circulation passage. Therefore, the medium temperature in the whole of the circulation passage, and that in the medium container are uniformalized with high accuracy.

As shown in step 202, it is judged whether the leading laboratory dish 21 reaches the dispensing position A or not. If the laboratory dish 21 reaches the dispensing position A, operations of steps 301 to 304 are conducted.

In step 301, the states of the control valves 18 and 19 are switched from the medium circulating state which is implemented in step 201, and the medium of a small amount remaining in the tip end of the nozzle 7 is discharged. Specifically, the first control valve 18 is opened and the second control valve is closed, and, after elapse of a predetermined time period, the dispensing pump 15 is stopped. At this time, the operation of the dispensing pump 15 is stopped while adjusting the switching timings of the control valves 18 and 19, whereby the amount of the discharged medium can be suppressed to a required minimum level.

In step 302, after the medium discharge for elimination of air bubbles and various bacteria is completed, the dispensing pump 15 is stopped, and the nozzle 7 is moved from the waiting position B to the dispensing position A.

In step 303, when the nozzle 7 is moved to the dispensing position A, the dispensing pump 15 is operated to pour an adequate amount of the medium 2 into the laboratory dish 21. This step constitutes the dispensing work.

Next, in step 304, after pouring of an adequate amount of the medium is ended, the dispensing pump 15 is stopped and the nozzle 7 is again returned to the waiting position B. After step 304 is ended, the control returns to the initial step 101.

It is a matter of course that the components of the medium dispensing apparatus are not restricted to those of the embodiment and their design may be suitably modified.

Next, the medium stirring and heat-retaining apparatus of an embodiment of the invention will be described in detail with reference to Figs. 3 and 4. Fig. 3 is an assembly view of the medium stirring and heat-retaining apparatus of the embodiment. In the embodiment, a medium 2 such as an agar medium is placed in a medium container 3 of high thermal conductivity, and a rod-like medium stirring rotor 5a in which both the ends are formed as magnetic poles is placed in the medium container 3. The medium stirring rotor corresponds to the first stirrer. While a pedestal 4 in which three legs 4c having a predetermined height are connected to a disk 4b wherein plural punched holes 4a are formed is placed on the side of the lower face of the medium container 3, the medium container 3 and the pedestal 4 are submerged in a heating bath 6a filled with a heat transfer fluid 6c (hereinafter, referred to as bath water) such as water or oil.

For example, the pedestal 4 is formed by stainless steel which is a nonmagnetic material, such as SUS3O4. The height of each leg 4c is set so that the bottom face of the medium container 3 is separated from that of the heating bath 6a by about 2 to 5 cm. When the height is too small, the pedestal makes contact with a bath water stirring rotor 5b (described later) in the heating bath 6a, to obstruct the stirring operation. By contrast, when the height is too large, the magnetic force due to a magnetic stirrer 11 is reduced, so that the turning force of the medium stirring rotor 5a is weakened. The punched holes 4a are formed in order to enhance the flowability of the bath water 6c, and improve the property of transferring heat to the medium container 3.

The heating bath 6a comprises: a ring heater 6 for heating the bath water 6c; a temperature sensor 6b which senses the temperature of the bath water; and a temperature adjusting device 8 which controls the heating operation of the ring heater 6 in accordance with an output signal of the temperature sensor 6b and a preset temperature which is input from the outside. The ring heater 6 is formed as a cartridge type which is detachable, so that the heater 6 can be easily detached as a single unit from the temperature adjusting device 8. The ring heater 6 and the temperature adjusting device 8 correspond to the heating means.

The body of the heating bath 6a is formed by stainless steel which is a nonmagnetic material, such as SUS304. A vacuum cavity may be formed in the peripheral wall of the heating bath 6a and the inner wall of the heating bath body serving as the heating bath bottom. In this case, the vacuum cavity can provide a heat insulation effect of reducing waste heat dissipation of the bath water 6c heated by the heater 6, to the outside of the bath, thereby improving the heat retaining property of the bath water 6c. By the vacuum cavity, moreover, the electric power which is consumed for heating by the temperature adjusting device 8 can be reduced as much as possible. The degree of vacuum in the vacuum cavity is about 10⁻⁵ Torr.

In the same manner as the medium container 3, a bath water rotor 10 for stirring is placed in the heating bath 6a. The bath water rotor 5b corresponds to the second magnetic stirrer.

As the rotors 5a and 5b, for example, commercially available ones having a diameter of 8 mm and a length of about 40 mm or smaller dimensions may be used. As the magnetic stirrer 5, for example, a commercially available one which can produce a magnetic field of 4,000 gauss may be used. It is a matter of course that it is preferable to use the apparatus with appropriately modifying the selection of the components in accordance with the capacities of the medium container and the heating bath, and the like.

An outlet port 3a is disposed in the medium container 3. A collecting pipe 12 which has a suction port 12a at the tip end is inserted through the outlet port 3a of the medium container 3. The collecting pipe 13 is connected to a dispensing pump which is not shown, so that a desired amount of the medium 2 can be supplied at a desired rate through the suction port 13a to the outside of the medium container 3 by the suction function of the dispensing pump.

Next, the stirring and heat-retaining operation by the stirring and heat-retaining apparatus will be described. Fig. 4 is a view illustrating the operation of the medium stirring and heat-retaining apparatus of the embodiment and partly showing the interiors of the medium container 3 and the heating bath 6a in the form of a section view.

First, the temperature adjusting device 8 senses the preset temperature which is input through a temperature set button 8a, and the bath water temperature in the heating bath 6a and detected by the temperature sensor 6b, and controls parameters such as the load current, the voltage, the load time period, and the like of the ring heater 6 and corresponding to the bath water temperature, whereby the bath water temperature is adequately adjusted. When the temperatures of the bath water and the medium balance with each other, the preset temperature is approximately equal to the medium temperature.

On the other hand, in the magnetic stirrer 5, the stirring rotation number relating to the rotation rates of the rotors 5a and 5b in the medium container 3 and the heating bath 6a is set through a rotation number setting dial 5c to, for example, about 200 to 400 rpm, and the medium 2 and the bath water 6c are then stirred. According to this configuration, heat of the ring heater 6 is transferred via the bath water 6c to the medium container 3 placed on the pedestal 4, without causing temperature unevenness, and further transferred from the medium container 3 to the medium 2. The magnetic stirrer 5 corresponds to the magnetic stirring means.

As described above, in the embodiment, the rotors 5a and 5b are disposed in the medium container 3 and the heating bath 6a, respectively, and the rotors 5a and 5b are rotated by the function of the magnetic stirrer 5, so that the interiors of the medium container and the heating bath are simultaneously stirred by the two rotors.

In the invention, as described above, the two baths (the medium container and the heating bath) are simultaneously stirred, whereby the temperature of the medium is managed with high accuracy. Because of the function of the simultaneous stirring, when the medium temperature is set to, for example, 49°C through the temperature set button 8a of the temperature adjusting device 8, the medium temperature in the whole of the interior of the medium container is uniformly held to 49 ± 1°C. In this way, a highly accurate control can be conducted in which the deviation of the medium temperature form the preset temperature can be reduced to about ± 1°C in any portion of the interior of the container. This enables the medium temperature to be stably set in a finer manner in a microbial test in which the temperature control has been usually recognized as to be difficult. As a result, even when viable cells enter a medium, the viable cells can live for a long time period.

When the collecting pipe 13 connected to the dispensing pump is inserted into the medium container 3, the medium which is uniformalized in temperature, viscosity, and the like can be directly supplied to the outside without taking out the medium container from the heating bath. Therefore, the medium can be continuously supplied in a stable state while the conditions such as the medium temperature and the viscosity are kept constant as much as possible.

The medium container 3 may be configured any container which can be immersed in the heating bath 6a. Therefore, a container having a shape other than a cylindrical shape, such as a bottle or a flask may be used as the medium container as far as it is not made of a magnetic material.

Furthermore, the capacity of the medium container is not limited. Even when the capacity is 500 cc or 2,000 cc, for example, stirring and heat retention can be conducted in the same manner as described above. In production of a medium, therefore, a medium of a large amount can be stably produced and maintained at one time. In the case where a medium of a required amount is to be produced in plural or about 5 or 6 batches, the production can be completed by a reduced number of or about 2 operations. Even when production is to be conducted in a further increased number of batches, it is possible to complete the production by a further reduced number of operations.

The medium viscosity can be further uniformalized by the stirring effect of the magnetic stirrer 5 and the rotor 5a, and the medium 2 can be prevented from being locally gelled.

The container support 4, the ring heater 6, the temperature sensor 6b, and the temperature adjusting device 8 can be easily attached to and detached from the heating bath 6a. Therefore, the cleaning work after the use of the apparatus can be simplified. Even in the case where sterility of water in the heating bath is important, such as a microbial test, management for sanitation can be easily performed. The medium container 3 can be easily attached and detached. Even when plural medium containers are to be sequentially heated (heat-retained), therefore, a medium container can be easily replaced with another one with a single operation.

When a medium container is formed as a transparent container made of glass or the like, the state of the contents of the container can be visually checked. Therefore, monitoring of a medium is made easy and a fine checking work can be conducted.

Another embodiment of the medium dispensing apparatus of the invention will be described with reference to the accompanying drawings.

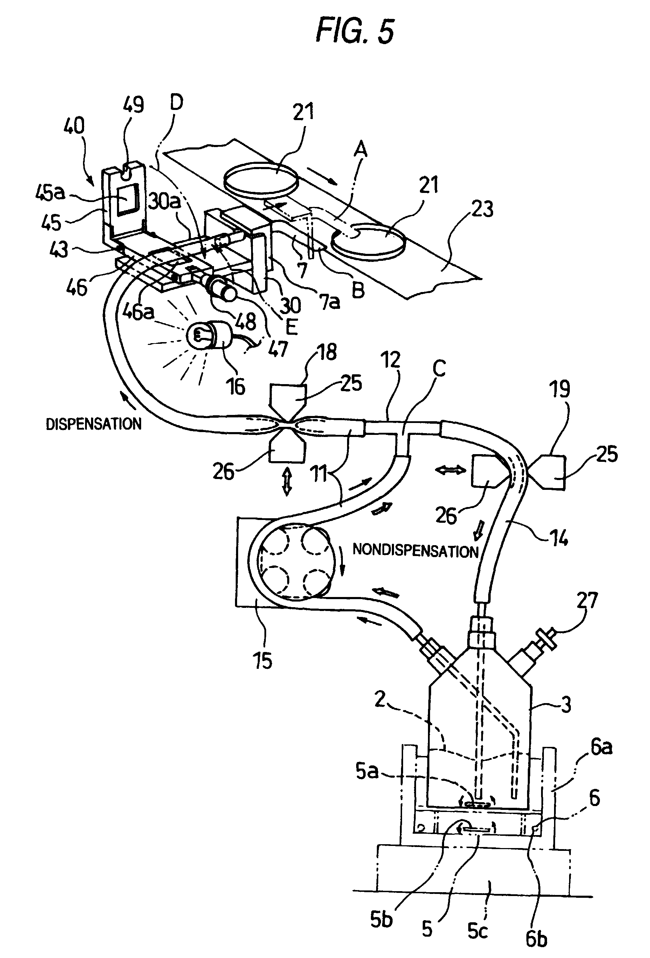

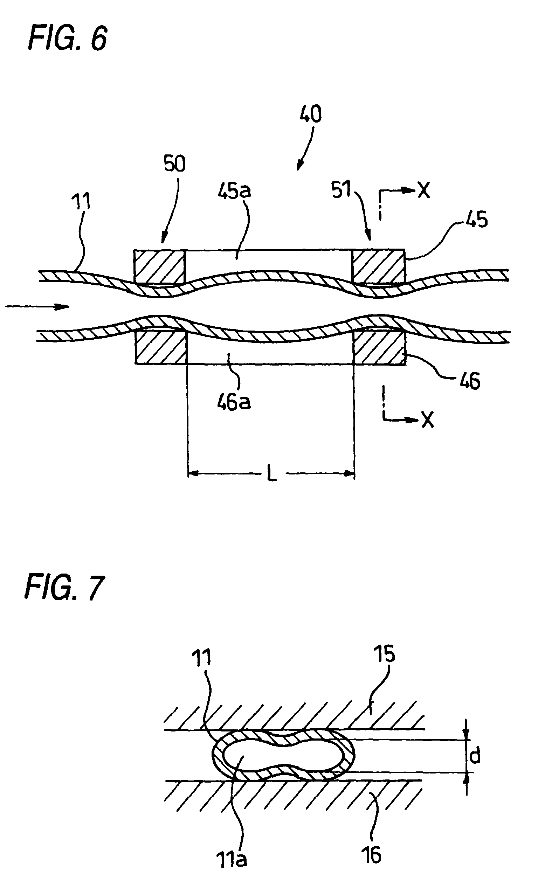

Fig. 5 is a schematic view of a second embodiment of the medium dispensing apparatus of the invention, Fig. 6 is a longitudinal section view taken along a liquid passage in a portion where pressing means shown in Fig. 5 is disposed, and Fig. 7 is a section view taken along the line X-X in Fig. 6.

Components which function in the same manner as those of the above-described medium dispensing apparatus 1 are designated by the same reference numerals, and their description is omitted.

The medium dispensing apparatus of the embodiment is configured in a substantially same manner with respect to the portion from the medium container 3 to the auxiliary heating means 16 in the medium dispensing apparatus shown in Fig. 1. Therefore, description of the portion is omitted.

The medium dispensing apparatus has at the tip end a dispensing nozzle 7 which supplies a specific amount of a medium to the predetermined container 21 such as a laboratory dish, and is configured so that the medium 2 accommodated in the medium container 3 is supplied to the dispensing nozzle 7. As shown in Figs. 5 to 7, the liquid supplying portion of the dispensing nozzle 7 is provided with pressing means 40 which forms throttle portions 50 and 51 (see Fig. 2) that reduce the diameter of the medium sending tube 11 at positions immediately before the nozzle 7.

The configuration of the embodiment is characterized in that, as shown in Fig. 6, the throttle portions 50 and 51 of a diameter which is smaller than that of another portion of the tube 11 are disposed in two positions of the tube 11.

For example, pressing means 40 comprising: an upper plate 45 having an opening 45a; and a lower plate 46 having an opening 46a sandwiches the tube 11 constituting the medium sending passage, so that the throttle portions 50 and 51 are formed in front of and in rear of the openings 45a and 46a.

As shown in Fig. 7, for example, the tube 11 serving as the medium sending passage is pressed by the upper plate 45 and the lower plate 46. Therefore, a section of each of the throttle portions 50 and 51 has a flat shape in which the maximum diameter d of a circular passage 11a that is about several millimeters in a usual state is reduced to several tenths.

In the pressing means 40, as shown in Fig. 5, for example, the lower plate 46 is attached to a fixing plate 30a of a holder setting table 30, and the upper plate 45 is swingably attached to the lower plate 46 via a hinge portion 43. An engaging pin 47 having a flange 48 is swingably attached to one end of the lower plate 46. On the other hand, a recess 49 which receives the engaging pin 47 is formed in one end of the upper plate 45.

As shown by the phantom lines in Fig. 5, when the upper plate 45 is closed (in the direction of the arrow D) while placing the tube 11 on the lower plate 46, and the engaging pin 47 is then swung (in the direction of the arrow E) so as to be engaged with the recess 49, the tube 11 can be sandwiched to form the throttle portions 50 and 51 (see Fig. 6) .

When it is configured so that the flange 48 of the engaging pin 47 is movable in the axial direction of the engaging pin (a configuration in which movement is enabled by screwing or the like), the distance between the upper and lower plates 45 and 46 is adjustable. In a preferred structure for adjusting the distance between the upper and lower plates 45 and 46, the upper plate 45 is urged in the closing direction (in the direction of the arrow D of the figure) by urging means (such as a torsion spring disposed in the hinge portion or the like) which is not shown.

For example, the dispensing nozzle 7 is fixed to the upper end side of the holder setting table 30 via a nozzle holder 7a. The container transporting means 23 is placed in the vicinity of the dispensing nozzle 7. The laboratory dishes 21 are continuously transported by the container transporting means 23.

The holder setting table 30 is configured so as to be moved in the anteroposterior direction by a driving cylinder which is not shown.

Next, description will be made with reference to the operation of the medium dispensing apparatus of the embodiment.

When dispensation is to be conducted by the abovedescribed medium dispensing apparatus, the laboratory dish 21 supplied from a container supply station (not shown) for a laboratory dish or the like is transported to a predetermined position (the region where the dispensing nozzle is placed) by the container transporting means 23.

With respect to the laboratory dish 21 which has been transported to the predetermined position, the lid of the laboratory dish 21 is taken off by a lid holding mechanism which is not shown, and a predetermined amount (about 5 cc) of the medium which previously contains viable cells is dispensed into the laboratory dish from the medium dispensing nozzle 7.

During this dispensation, the second control valve 19 is set to be in the closed state, and the first control valve 18 is opened and closed, thereby enabling only the predetermined amount of the medium 2 to be supplied. As described above, when the medium 2 is supplied from the dispensing nozzle 7 to the predetermined laboratory dish 21, the tube 11 which is a liquid passage from the pump 15 serving as the liquid supply driving means to the dispensing nozzle 7 is in a hermetically sealed state. Since the diameter of the tube 11 is reduced in two positions of a midway of the tube 11, no liquid dripping occurred and excellent liquid cutting was attained.

Although the pump 15 produces pulsation when a liquid is supplied, the pulsation of the pump 15 was not produced in the medium ejection from the dispensing nozzle 7.

In the second embodiment of the medium dispensing apparatus of the invention, the configuration which is substantially identical with the portion from the medium container 3 to the auxiliary heating means 16 in the medium dispensing apparatus shown in Fig. 1 is employed. The liquid supply mode in the second embodiment is not restricted to this, and the second embodiment can be effectively applied to a liquid supply mode in which pulsation is produced in a liquid flow passing through a nozzle.

With respect to configurations in which the throttle portion is formed in three or four positions of the tube 11 as another embodiment of the medium dispensing apparatus of the invention, particularly the liquid supplying section, dispensation tests were conducted in the strictly same conditions.

As a result, also in these cases, no liquid dripping occurred, pulsation of the medium ejection was not produced, and the same effects as those of the first embodiment were attained.

As comparative tests, dispensation tests were conducted on cases where the tube 11 has one throttle portion or no throttle portion.

In these tests, the dispensation tests were conducted under the strictly sane conditions except the throttle portion.

As a result, in both the cases where one throttle portion is formed and no throttle portion is formed, liquid dripping and pulsation in the medium ejection occurred.

In the dispensing apparatus of the invention, the medium passage such as the medium sending passage is configured by a silicone tube. The invention is not restricted to this. Also the control valves and the pump may be variously modified.

Also the distance (L) of the throttle portions is not particularly restricted, and may be adequately set.

### Industrial Applicability

As described above, according to the medium dispensing apparatus and the dispensing method of the invention, the whole of the medium sending passage from the medium container to the branching position of the medium returning passage, and the medium returning passage is adjustingly heated to an adequate temperature by the medium which is in the medium container and which is circulated during waiting for dispensation, and the medium sending passage from the branching position of the medium sending passage with respect to the medium returning passage to the nozzle is adjustingly heated by the auxiliary heating means. Therefore, the whole of the medium passage which guides the medium from the medium container to the nozzle is held to be in a uniform temperature state where the medium temperature is not substantially lowered, and hence the temperature of the medium can be stably maintained to an adequate temperature with high accuracy.

Moreover, the outside air in an axenic condition which has been filtered by a filter is supplemented into the medium container by an amount corresponding to the volume of the medium which is reduced as a result of dispensation. Therefore, the air which makes contact with the medium in the medium container can be always kept to be in an axenic condition. Consequently, the whole of the interior of the medium passage can be sterilized.

The medium dispensing apparatus of the invention comprises the nozzle which is extendable and retractable between the predetermined dispensing position A and the waiting position B separated from the dispensing position A, and the controller has a function of controlling the operation of moving the nozzle. In this case, in the medium dispensing method, a state where the nozzle is retracted to the waiting position B is held, and, immediately before a start of the dispensing operation, the dispensing pump is operated for a predetermined time period under a state where the second control valve is closed and the first control valve is opened, whereby a medium which is caused to be inadequate in quality by residue of the medium in the nozzle and the medium sending passage is discharged at the waiting position B.

Even in the course of delivery of the medium by the dispensing pump, therefore, the operation of disposing the medium can be instantly stopped without obstructing the operation of the dispensing pump, by closing the first control valve and opening the second control valve. The amount of a medium which is to be discharged in order to purge air or dispose a medium that has been contacted with the outside air can be finely adjusted. By disposal of a minimum amount of a medium, dispensation of a medium which is made inadequate in quality can be surely prevented from occurring. Therefore, the quality of a medium which is poured into the dispensing container in dispensation can be kept to be uniform and excellent at a high level.

According to the invention, the rotors are disposed in the medium container and the heating bath, respectively, and simultaneously rotated. Even in a simple configuration, therefore, the interiors of the medium container and the heating bath can be sufficiently stirred so as to uniformalize the heat distribution, and the heat transfer from the heating bath to the medium container can be stably conducted. As a result, unevenness of the medium temperature is reduced, and the medium temperature can be controlled with high accuracy. Since a medium is stirred, the medium can be kept in a more uniform state.

In the medium dispensing apparatus and the dispensing method of the invention, in the medium sending passage, throttle portions of a diameter which is smaller than a diameter of another portion of the medium sending passage are disposed in two or more positions. Even for a medium which easily drips, such as an agar medium, therefore, liquid dripping from the dispensing nozzle can be completely avoided, the medium can be correctly supplied, and production of bubbles during dispensation of the medium can be eliminated.

Therefore, nonuniformity of test conditions due to reduction of the test accuracy and uneven supply of a medium which are caused by production of bubbles can be avoided, and hence the test accuracy and the reliability can be improved.

## Claims

1. A medium dispensing apparatus which pours a medium 2 stored in a medium container 3 having a stirring and heat-retaining apparatus into a dispensing container 21, said medium dispensing apparatus comprises:
a medium sending passage 11 serving as a medium passage which guides the medium 2 in the medium container 3 to a nozzle 7 for pouring the medium into said dispensing container 21;
a medium returning passage 14 serving as a medium passage in which one end is branched from a midway of said medium sending passage 11, and the other end is connected to said medium container 3 to return the medium 2 to the medium container 3;
a dispensing pump 15 which is disposed upstream from a branching position C between said medium sending passage 11 and said medium returning passage 14, and which sends out the medium 2 in said medium container 3 to said medium sending passage 11;
a first control valve 18 which is disposed in a midway of said medium sending passage 11 and downstream from the branching position C, and which opens and closes the passage of said medium sending passage 11;
a second control valve 19 which is disposed in a midway of said medium returning passage 14 and downstream from the branching position C, and which opens and closes said medium returning passage 14;
auxiliary heating means 16 for, in a zone which is downstream from the branching position C of said medium sending passage 11, adjustingly heating the medium in said medium sending passage 11 to a desired temperature; and
a controller which controls operations of said dispensing pump 15, said auxiliary heating means 16, and said first and second control valves 18 and 19.

2. The medium dispensing apparatus according to claim 1, wherein said auxiliary heating means 16 is an infrared lamp.

3. The medium dispensing apparatus according to claim 1 or 2, wherein a filter 27 which makes air to be supplemented into said medium container 3 into an axenic condition is disposed at an upper position of said medium container 3.

4. The medium dispensing apparatus according to claim 1, wherein, in said medium sending passage 11 which is downstream from said first control valve 18, throttle portions 50 and 51 of a diameter which is smaller than a diameter of another portion of said medium sending passage 11 are disposed in two or more positions.

5. The medium dispensing apparatus according to claim 4, wherein at least a portion of said medium sending passage 11 is made of an elastic material, and the portion made of the elastic material is sandwiched by pressing means 40, whereby said throttle portions 50 and 51 are formed.

6. The medium dispensing apparatus according to claim 5, wherein said pressing means 40 is configured so that throttle diameters of said throttle portions 50 and 51 are adjustable.

7. The medium dispensing apparatus according to claim 1 or 4, wherein said medium stirring and heat-retaining apparatus comprises: a heating bath 6a which stores a liquid heat transfer medium in which said medium container 3 is to be immersed, said bath having heating means 6 for heating the heat transfer medium to retain the temperature of the medium in said medium container 3; a pedestal 4 on which said medium container 3 is placed in said heating bath 6a; a first stirrer 5a which is immersibly disposed for stirring the medium in said medium container 3; a second stirrer 5b which is submergedly disposed on a bottom face of said heating bath 6a; and magnetic stirring means for rotating simultaneously said first stirrer 5a and said second stirrer 5b, said pedestal 4 being disposed at a height which is above said second stirrer 5b and at which rotation of at least said second stirrer 5b is not obstructed.

8. A dispensing method which uses the medium dispensing apparatus according to claim 1 or 4 to pour a predetermined amount of the medium into said dispensing container, wherein,
during an operation of waiting for dispensation, said first control valve 18 is closed, and said second control valve 19 is opened; in said medium sending passage 11 from said medium container 3 to the branching position C, and said medium returning passage 14, the medium 2 is circulated by said dispensing pump 15 to hold the medium to an adequate temperature; and, in said medium sending passage 11 from the branching position C to said nozzle 7, the medium 2 is held to an adequate temperature by heating conducted by said auxiliary heating means 16.

9. A dispensing method which uses said medium dispensing apparatus according to claim 1 or 4 to pour a predetermined amount of the medium into said dispensing container, wherein,
during an operation of waiting for dispensation, a state where said nozzle 7 is retracted to a waiting position B separated from a dispensing position A is held; and, at a start of a dispensing operation, said dispensing pump 15 is operated for a predetermined time period immediately before the start under a state where said second control valve 19 is closed and said first control valve 18 is opened, to discharge a medium which is at a tip end of said nozzle 7 and inadequate in quality, at the waiting position B, and said nozzle 7 is thereafter advanced to the predetermined dispensing position A to conduct dispensation.

10. A dispensing method which uses said medium dispensing apparatus according to claim 3 to pour a predetermined amount of the medium into said dispensing container, wherein,
in said medium sending passage 11 from said medium container 3 to the branching position C, and said medium returning passage 14, during an operation of waiting for dispensation, the medium 2 in said medium container 3 is circulated by said dispensing pump 15 while said first control valve 18 is closed and said second control valve 19 is opened, to hold the medium 2 in an axenic condition; and, during dispensation of the medium 2, air in an axenic condition is supplemented into said medium container 3 via said filter 27.

11. A medium dispensing apparatus comprising:
a dispensing nozzle 7 which supplies a specific amount of a medium 2 to a predetermined container;
liquid supply driving means for sending the medium 2 to said dispensing nozzle 7;
a medium sending passage 11 which forms a hermetically sealed liquid passage between said dispensing nozzle 7 and said liquid supply driving means; and
valve means for opening and closing said medium sending passage 11 in order to control an amount of the medium 2 supplied to said container,
wherein throttle portions 50 and 51 of a diameter which is smaller than a diameter of another portion of said medium sending passage 11 being disposed in two or more positions of said medium sending passage 11.

12. A medium dispensing apparatus according to claim 11, wherein at least a portion of said medium sending passage 11 is made of an elastic material, and the portion made of said elastic material is sandwiched by pressing means 40, whereby said throttle portions 50 and 51 are formed.

13. A medium dispensing apparatus according to claim 12, wherein said pressing means 40 is configured so that throttle diameters of said throttle portions 50 and 51 are adjustable.

14. A dispensing method which uses said medium dispensing apparatus according to claim 11, wherein,
when the medium is to be supplied from said dispensing nozzle 7 to a predetermined container by supplying a liquid by said liquid supply driving means, and opening and closing said control valve means,
the medium is supplied while said medium sending tube 11 serving as a liquid passage from said liquid supply driving means to said dispensing nozzle 7 is set to be in a hermetically sealed state, and throttling portions 50 and 51 which reduce a diameter of said medium sending tube 11 are formed in at least two positions in a midway of said medium sending tube 11.

15. A medium stirring and heat-retaining apparatus comprising:
a heating bath 6a which stores a liquid heat transfer medium in which a medium container 3 containing a medium is to be immersed;
heating means 6, disposed in said heating bath 6a, for heating the heat transfer medium to retain a temperature of the medium in said medium container 3;
a pedestal 4 on which said medium container 3 is placed in said heating bath 6a;
a first stirrer 5a which is immersibly disposed for stirring the medium in said medium container 3;
a second stirrer 5b which is submergedly disposed on a bottom face of said heating bath 6a; and
magnetic stirring means for rotating simultaneously said first stirrer 5a and said second stirrer 5b,
wherein said pedestal 4 being disposed at a height which is above said second stirrer 5b and at which rotation of at least said second stirrer is not obstructed.
